# EUROPEAN PATENT APPLICATION

(11) **EP 0 898 962 A1**
(43) Date of publication of application: **03.03.1999**
(21) Application number: 98306942.8
(22) Date of filing: 28.08.1998
(51) Int. Cl.: A61K 9/70, A61K 38/04, A61K 47/00, A61K 47/02, A61K 47/04, A61N 1/20, A61N 1/30

(54) **Transdermal patches and methods for inactivating skin proteases**

(30) Priority: 28.08.1997 US 919829
(71) Applicant: Becton, Dickinson and Company, Franklin Lakes, New Jersey 07417-1880 (US)
(72) Inventor: Down, James Arthur, Cary, NC 27511 (US); Williams, Amy Casey, Huntington Woods, MI 48070 (US)
(74) Representative: Ruffles, Graham Keith

(57) **Abstract**

Methods of inactivating skin proteases in order to permit the transdermal delivery of peptide drugs, specifically via iontophoretic patches, are described. In general, the methods involve inhibiting one or more proteases in a patient's stratum corneum by using an agent which reacts with histidine residues within the proteases. Specific examples of such methods include use of compounds which are specifically reactive with histidine residues in proteins; use of iodine containing compounds; use of hydrogen peroxide; use of indole reactive compounds; use of ultra-violet light; use of disulfide reducing reagents; controlling pH; use of zinc in conjunction with high pH; and use of oxidizing compounds. Transdermal patches including agents which react with histidine residues in skin proteases are also described.

## Description

### BACKGROUND OF THE INVENTION

### Field of the Invention

The invention relates to the iontophoretic transdermal delivery of drugs, specifically peptides.

### Skin Proteases and Iontophoresis of Peptide Drugs

Polypeptides with specific pharmacological properties are part of an expanding repertoire of new drugs. For many peptide drugs, this results from their relatively recent discovery and simultaneous accessibility through the development of recombinant DNA technology. Because they are natural endogenous mediators, peptides have a potential advantage over small molecule pharmaceutics through potent efficacy, and low levels of side effects. Transdermal iontophoresis offers an advantageous route for some peptide drugs due to patient convenience and dosage compliance. The noninvasive nature of iontophoretic delivery may provide value by increasing patient comfort, accepting of the dosage and reduce health risk from blood borne pathogens.

However, there appear to be two main barriers to transdermal iontophoretic peptide delivery. Ionic charge of a peptide at the pH of skin during iontophoresis as well as ionic, hydrophobic or biological interactions between a peptide and components of the skin may reduce its mobility. Secondly, peptide drugs may be proteolytically degraded by skin proteases during transdermal delivery.

Effects of proteases on in vivo or in vitro transdermal delivery has been reported for vasopressin and vasopressin analogues (Morimoto et al., Int. Journal Pharmaceutics 81: 119-125, 1992), des-enkephalon-γ-endorphin (Bodde et al., Biochem. Soc. Trans. 17: 943-945, 1989), leucine enkephalin (Choi at al., 1990), and salmon calcitonin (SCT) (Morimoto et al., J. Pharm. Pharmacol. 44; 216-218, 1992). This was concluded because addition of protease inhibitors appear to enhance delivery as determined by increased biological effect of the peptides. For example, camostat mesilate (a serine protease inhibitor) increased the hypocalcemic effect of iontophoretically-delivered SCT (Morimoto, J. Pharm. Pharmacol. 1992) and the antidiuretic effects of vasopressin delivered iontophoretically in rats (Morimoto, Int. Journal Pharmaceutics, 1992). We have observed that incubation of insulin analogues or SCT on human skin results in degradation, which supports the notion that proteolysis accounts for previously observed losses of these peptide drugs during iontophoretic delivery.

Each peptide drug is characterized by having a unique amino acid sequence. Proteases function by recognizing specific amino acids, amino acid classes or amino acid sequences and cutting the polypeptide chain at that site. Consequently, the susceptibility of a peptide drug to degradation by skin proteases will depend on its specific sequence. The substrate specificity of SCCE (as described below, an enzyme found in human skin, is rather broad; requiring an aromatic amino acid in the P1 position of the substrate binding site for substrate cleavage (Asa Skytt et al, Biochem. Biophys. Res. Comm. 211:586-589 (1995)). Therefore, the susceptibility of individual peptides to skin proteases has been determined empirically. To ensure that iontophoretically-delivered peptide drugs are not degraded by skin proteases, protease inhibition strategies are required :

Several proteases have been reported in skin components and cells. Stratum corneum, the outermost layer of the skin, contains two proteases of about 25,000-29,000 Daltons size (Egelrud, J. Investigative Dermatology, 101:200-204, 1993; Egelrud and Lundstrom, Acta. Derm. Venereol. (Stockh) 71: 471-474, 1991; Suzuki et al., Arch. Dermatol. Res. 286: 249-253, 1994). Because these enzymes show the same activity and enzymatic specificity it is uncertain whether they are distinct proteins or whether they are variants of the same one. However, their enzymatic specificity is similar to chymotrypsin, a well characterized digestion enzyme and thus Egelrud et al. have named the protease(s) "stratum corneum chymotrypsin-like enzyme" (SCCE).

SCCE is located in the outer layer of the skin and research suggests that this layer may present a significant barrier to transdermal delivery of some peptides. The reduced mobility of peptides through stratum corneum will increase their contact time with SCCE. Thus, it is probable that these enzymes are directly responsible for proteolytic degradation of peptides administered by passive and iontophoretic patches.

There are prior patents showing the use of protease inhibitors to inhibit proteases (see e.g. U.S. 5,091,193, a treatment for diaper rash including zinc oxide, physic acid and optionally an antienzyme, such as antilipase; U.S. 5,008,242, treatment of inflammation, using α-1-antichymotrypsin; U.S. 5,134,119, treatment of inflammation using a substituted α-antitrypsin; U.S. 4,906,457, a topical composition for reducing the risk of skin cancer, including "inhibitors" of various proteases). Other examples are U.S. 5,244,673; U.S. 5,183,900; U.S. 5,290,762; U.S. 5,239,078; U.S. 3,950,509; U.S. 5,306,444; U.S. 5,284,829; U.S. 5,252,560.

However, use of the methods described in the prior art suffer from many drawbacks. For example, many conventional protease inhibitors are extremely toxic and therefore unsuitable for use in a transdermal drug delivery system.

Further, polypeptide protease inhibitors such as soybean trypsin inhibitor or sprotinin are too large to enter the skin by methods such as iontophoresis.

Protease inhibitors that are not immediately poisonous have uncertain or unknown toxicology.

Some protease inhibitors are very unstable and quickly lose their ability to inhibit. Examples are nafamostat mesylate, camostat mesylate, and chloromethyl ketones.

Further, use of broad spectrum protease inhibitors may lead to unwanted inhibition of other skin enzymes, and consequently unwanted changes in skin metabolism.

The present invention discloses several ways to inhibit skin proteases to thereby permit transdermal delivery of peptide drugs. The compounds used in the methods of this invention are small enough to enter the skin. Many of the inhibiting agents have been used in other applications, and are therefore known to be non-toxic , such as iodine, UV light, zinc oxide and pH adjustment. These compounds are not broad spectrum protease inhibitors and therefore should not interfere with normal skin metabolism.

### SUMMARY OF THE INVENTION

The present invention discloses several ways of inactivating skin proteases to thereby permit transdermal iontophoretic delivery of drugs. These methods are found in ten specific embodiments:
1. Use of compounds which are specifically reactive with histidine residues in proteins;
2. Use of iodine-containing compounds;
3. Topical application of iodine solution;
4. Use of hydrogen peroxide;
5. Use of indole-reactive compounds;
6. Use of ultra-violet light;
7. Use of disulfide-reducing reagents;
8. Control of pH;
9. Use of Zinc in conjunction with a high pH; and
10. Use of oxidizing compounds,
Embodiments of each of the above methods are exemplified herein.

### DETAILED DESCRIPTION OF THE INVENTION

The present invention includes ten different ways to inhibit skin proteases from interfering with the transdermal, iontophoretic delivery of peptide drugs.

As used herein, a peptide will be referred to as a short sequence of amino acid residues (usually less than about 30). Unless held rigid by an internal bond, such as a disulfide, peptides usually do not retain a unique, stable structure in water. The term polypeptide is usually used to refer to proteins, which are longer amino acid polymers with stable, unique three-dimensional structure (usually 100 residues or more). For present purposes, the terms peptide and polypeptide will be used synonymously.

Peptidases are enzymes that cut peptides into their constituent amino acid components and likewise proteinases are enzymes that cut proteins into peptide fragments. Since many of such enzymes perform both functions, the generic term "protease" will be used to describe either type. Proteases can be further differentiated according to whether they remove amino acid residues sequentially from the ends of peptides or proteins (i.e. "exoproteases" such as aminopeptidase or carboxypeptidase) or they produce cleavage within a polypeptide sequence to produce two separate peptide fragments (i.e. "endoproteases" such as trypsin or chymotrypsin). The term "protease" will be used here to describe either type of enzyme.

The construction of the iontophoretic patch used is not critical in the present invention. In presently known iontophoretic devices such as those, for example, disclosed in U.S. Patent Nos. 5,645,526 (Flower), 5,540,669 (Sage, Jr. et. al.), 5,498,235 Flower), 5,458,569 (Kirk, III et. al.), and 5,256,137 (Sage, Jr.), the disclosures of which are hereby incorporated by reference. Typically, such devices use at least two electrodes, and both of these electrodes are positioned to be in intimate electrical contact with some portion of the skin of the body. One electrode, called the active or donor electrode, is the electrode from which the ionic substance, medicament, drug precursor or drug is delivered into the body by iontophoresis. The other electrode, called the counter or return electrode, serves to close the electrical circuit through the body. In conjunction with the patient's skin contacted by the electrodes, the circuit is completed by connection of the electrodes to a source of electrical energy, e.g., a battery. For example, if the ionic substance to be delivered into the body is positively charged (i.e., a cation), then the anode will be the active electrode and the cathode will serve to complete the circuit. If the ionic substance to be delivered is negatively charged (i.e., an anion), then the cathode will be the active electrode and the anode will be the counter electrode.

Alternatively, both the anode and cathode may be used to deliver drugs of opposite charge into the body. In such a case, both electrodes are considered to be active or donor electrodes. For example, the anode can deliver a positively charged ionic substance into the body while the cathode can deliver a negatively charged ionic substance into the body.

It is also known that iontophoretic delivery devices can be used to deliver an uncharged drug or agent into the body. This is accomplished by a process called electroosmosis. Electroosmosis is transdermal flux of a liquid solvent (e.g., the liquid solvent containing the uncharged drug or agent) which is induced by the presence of an electric field imposed across the skin by the donor electrode. As used herein, the terms "iontophoresis" and "iontophoretic" apply equally to electrically powered devices which deliver charged/ionic agents by iontophoresis as well as to electrically powered devices which deliver uncharged/nonionic agents by electroosmosis.

The iontophoretic delivery device of the present invention is preferably flexible enough to conform to contours of the body. While not limited to any particular size or shape, the device typically is about two or three inches long, about one and one-half inches wide, and has a thickness of approximately one-quarter of an inch. The combined skin-contacting areas of electrode assemblies can vary from less than 1 cm² to greater than 200 cm². The average device however, will have electrode assemblies with a combined skin-contacting area within the range of about 5 to 50 cm². As constructed, electrode assemblies are electrically isolated from each other until the device is applied to the human body, whereupon a circuit through the human tissue is completed between the electrode assemblies.

A matrix of reservoirs to hold the drug, or beneficial agent, and the "counter" ion is provided. A reservoir can be any material adapted to absorb and hold a sufficient quantity of liquid therein in order to permit transport of agent therethrough by iontophoresis. For example, gauzes made of cotton and other absorbent fabrics as well as pads and sponges, both natural and synthetic, may be used. Most preferably, the matrix of reservoirs is composed, at least in part, of a hydrophilic polymer material. Both natural and synthetic hydrophilic polymers may be used. Suitable hydrophilic polymers include polyvinylpyrrolidones, polyvinyl alcohol, polyethylene oxides such as Polyox^{®} manufactured by Union Carbide Corp.; Carbopol^{®} manufactured by BF Goodrich of Akron, Ohio; blends of polyoxyethylene or polyethylene glycols with polyacrylic acid such as Polyox^{®} blended with Carbopol^{®}, polyacrylamide, Klucel^{®}, cross-linked dextran such as Sephadex (Pharmacia Fine Chemicals, AB, Uppsala, Sweden) ; Water Lock^{®} (Grain Processing Corp., Muscatine, Iowa) which is a starch-graft-poly(sodium acrylate-coacrylamide)polymer; cellulose, derivatives such as hydroxyethyl cellulose, hydroxypropylmethylcellulose, low substituted hydroxypropylcellulose, and cross-linked Na-carboxymethylcellulose such as Ac-Di-Sol (FMC Corp., Philadelphia, Pa.) ; hydrogels such as polyhydroxyethyl methacrylate (National Patent Development Corp.); natural gums, chitosan, pectin, starch, guar gum, locust bean gum, and the like, along with blends thereof. Of these, polyvinylpyrrolidones are preferred.

In order to conduct electrical current, the reservoirs must be sufficiently hydrated to allow ions to flow therethrough. In most cases the liquid used to hydrate the matrices of the reservoirs will be water, but other liquids including non-aqueous liquids, can also be used to "hydrate" (i.e., activate) the matrices of the reservoirs. In the typical case where the hydrating liquid is water, the matrices of the reservoirs will be at least partly composed of a hydrophilic material such as a hydrophilic polymer, a cellulose sponge or pad or other water retaining material. Most preferably, the matrices of the reservoirs will be at least partly composed of a hydrophilic polymer of the type described hereinbefore.

The beneficial agent or drug, in the case of the donor electrode reservoir and the electrolyte salt in the case of the counter electrode reservoir may be added to the reservoir matrix either at the time of manufacture of the device or at the time of use of the device. For example, when the drug or electrolyte is added to the reservoir matrix at the time of manufacture of the device, blending of the drug or electrolyte with the reservoir matrix components can be accomplished mechanically either by milling, extrusion, or hot-melt mixing. The resulting dry state reservoirs may then be prepared by solvent casting, extrusion or by melt-processing, for example. In addition to the drug and electrolyte, the reservoirs may also contain other conventional materials such as dyes, pigments, inert fillers, and other excipients.

On the other hand, the reservoirs may be manufactured with no drug or electrolyte. In such a case, the drug and electrolyte can be added to the reservoirs, by adding a solution of the drug and electrolyte to the appropriate reservoir matrix at the time of use.

Primary requirements of a suitable drug are that it be charged or capable of modification to carry a charge. Appropriate selection of drugs for iontophoretic applications include selection based on specific conductivity (i.e., estimates how easily drugs move in solution when an electric current is applied).

Modification of drugs for iontophoretic delivery is guided by well-known procedures. For example, to deliver a positively charged drug, the chloride or hydrochloride form of the drug can be made and placed in the iontophoretic device reservoir for delivery. General texts in the field include *Remington's Pharmaceutical Sciences,* Ed. Arthur Osol, 16th ed., 1980, Mack Publishing Co., Easton, Pa. Typically the basic (OH- or amine) or acid (H⁺) form of the active agent is made, depending on whether the anionic (negative charged ion) or cationic (positive charged ion) form of the active agent is to be delivered. Likewise, the composition is typically dissolved in a suitable solvent to obtain the ionic from for iontophoretic delivery. Suitable solvents include polar liquids such as water, glycerine, and lower alkyl alcohols such as methyl alcohol, ethyl alcohol, and branched alcohols such as isopropyl alcohol.

In this invention the effective amount of a drug means that amount needed to produce the intended result following its iontophoretic administration. The effective amount will vary, depending, among other factors, on the physiological effect as determined by the serum level of desired drug, rate of clearance of the drug, and intradermal metabolism desired.

By studying the biochemical characteristics of proteases extracted from human stratum corneum it was discovered that the active site of the enzyme has a reactivity to reagents specific for the amino acids histidine or cysteine. Moreover, reaction with such reagents inhibited the stratum corneum proteases. Since such reagents do not necessarily inhibit enzymes, the result that they inhibited the proteases was unforeseen and represented a new strategy for stopping the enzymatic activity of the skin proteases.

Stratum corneum-chymotryptic enzyme (SCCE) has been shown to be inhibited by zinc sulfate (Egelrud, 1993; Lundstrom and Egelrud, 1991) but the importance of this is not known. We confirmed on zymograms that SCCE was inhibited by every zinc compound we tested: zinc chloride, zinc acetate, zinc oxide and zinc nitrate. Phipps (U.S. Patent 5,221,254) has claimed use of divalent cations such as zinc for reducing sensation during iontophoresis. However, we tested mercury and cadmium, which have chemical properties very similar to zinc, to determine whether the inhibition could be induced by other metals and found that indeed only zinc stopped SCCE activity.

The previous findings suggested a unique role for zinc in control of SCCE. We hypothesized that SCCE may be like elastase, collagenase and other matrix metalloendoproteinases which use active-site bound metal ions to catalyze proteolysis. However, incubation with various metals (Fe⁺⁺, Fe⁺⁺⁺, Cu⁺⁺, Co⁺⁺, Ba⁺⁺, Cs⁺⁺, Ni⁺, Ca⁺⁺, Mg⁺⁺, Mn⁺⁺) did not reduce or enhance activity as might be expected if they displaced or replaced a bound catalytic metal ion. Also, incubation with numerous metal chelators (EDTA, EGTA, 1,10 phenanthrolene, chelex) did not stop proteolytic activity. These results argued against SCCE being a metaldependent protease.

SCCE proteolytic activity was observed across a pH range of 4 to 9 with maximum at pH 6. However, in the presence of zinc chloride activity was seen at pH's of 6 and below. It should be noted that the previous experiments were performed under published standard conditions at pH 7. These results offered insight into the zinc-induced inhibition of SCCE. Because histidines in proteins have a pKa of about 6.5 and in the unprotonated state (above pH 6.5) chelates divalent metal ions such as zinc, (Biophysical Chemistry Part 1: Conformation of Biological Molecules C. Cantor, P. Schimmel W.H. Freeman & Co. 1980), we hypothesized that SCCE contained a reactive histidine which coordinated zinc with another residue (histidine or cysteine) within the activate site to thereby inactivate the enzyme. Such a mechanism has been developed artificially to control enzymatic activity in recombinant proteases, but we are not aware of any natural proteins demonstrating such a control mechanism. Support for this idea came from the fact that SCCE was inhibited by low concentrations (<1mM) of dimethyl pyrocarbonate, a compound that specifically modifies histidine residues.

It should be noted that dimethyl pyrocarbonate is toxic and probably could not be put safely into a transdermal patch. However, these results suggest that any compound which reacts with histidine amino acid residues in proteins should be able to react with the stratum corneum enzymes and inhibit them.

The reactivity of a chemical reagent with an amino acid depends on factors such as pH, residue accessibility, its microenvironment within the protease as well as the concentration of the chemical reagent. Therefore, the actual reactivity of the amino acid residues in proteins and peptides must be empirically determined. As shown in the following table, a variety of chemical reagents were used to delineate the chemical reactivity of SCCE. Except in the case of dimethyl pyrocarbonate, the inhibition noted may be due to an effect on one or numerous residues or residue types.

These experiments were done by incubating stratum corneum extracts with the chemical reagent for 30 minutes at 37° prior to a zymogram analysis. The fact that in each case the inhibition remained during the zymogram analysis indicates they were permanent, covalent modifications. Iodine solution, in the form of Povidone, was also tested by topical application to human skin prior to tape stripping and extraction of SCCE. Povidone is a 1% solution of iodine that is used as a surgical disinfectant and is commercially available.

| | Chemical reactivity^{**} | Inhibitory effect |
|---|---|---|
| Reagent | | |
| Iodine (Povidone) | histidine, tyrosine, cysteine | + |
| | | |
| Iodogen | cysteine, methionine, histidine, tryptophan | + |
| Chloramine T | cysteine, methionine, histidine, tryptophan | + |
| | | |
| Dimethyl pyrocarbonate | histidine > > tyrosine | + |
| Hydrogen peroxide | cysteine, methionine | + |
| | | |
| Hydrogen peroxide + Fe^{∗∗} | cysteine, methionine, histidine | + |
| Dithiothreitol | cysteine | + |
| | | |
| Cysteine | cysteine | + |
| N-aceryle cysteine | cysteine | - |
| | | |
| acetic anhydride | lysine, cysteine, tyrosine, histidine | + |
| UV light (photooxidation) | histidine, cysteine >tyrosine | + |
| | | |
| N-acetyl imidazole | histidine, tyrosine, cysteine > lysine | - |
| N-bromosuccinimide | cysteine > tyrosine > histidine | + |
| | | |
| Succinic anhydride | lysine, cysteine > tyrosine,histidine | |

| | | |
|---|---|---|
| ** G. E. Fears and R. E. Feeney, 1971, Chemical Modification of Proteins, Holden-Day Inc , San Franscisco. | | |

These results supported the idea that compounds which react with histidine or reduce cystine disulfide bonds will produce inhibition of the stratum corneum chymotryptic enzyme.

### Skin Proteases

In each experiment described in the following Examples, skin proteases were obtained by using 3M book tape #845 in strips which were approximately 7x4 cm. The strips were placed on the volar forearm of a human subject and smoothed, then peeled off. This process was repeated up to six times on the same skin area, until the tape no longer adhered to the skin. The tape was then extracted with sodium dodecyl sulfate, SDS.

To eliminate the possibility that the effects shown in the experiments were a consequence of a protease structure specific to an individual, and that within the population there might be different enzymes that would be responsible for peptide degradation, tape extractions were obtained from 36 individuals of varying age, sex and color. Zymogram profiles were obtained for these samples. The profiles showed enzymatic activity at identical molecular weight and similar intensity, thus suggesting the enzyme under study is common throughout the population. Accordingly, the inhibitory agents described in the Examples should have broad applicability.

### Protease Extraction

Protease was extracted from the tape strip sample by placing each tape strip in a disposable Falcon tube and received 100 µl of 1% SDS in 50 mM sodium phosphate at pH 8.0. The tube was then rocked or rotated for 60 minutes, then centrifuged at 1,000 rpm for five minutes. The fluid extracts were aspirated and used immediately, or they were stored at -20° C until use.

### Zymograms

Zymograms were then conducted using the eluates and the agent under consideration. Zymograms are gels that can be used to detect proteases. The Zymogram gel consists of 12% polyacrylamide gel with 0.2% α casein incorporated as a substrate. Proteases are characterized as clear bands against a dark blue background. Proteases with molecular weights in the approximate range of 30-200KDa can be characterized with a 12% zymogram gel.

Stock zymogram gel solution was made as follows:
100 ml acrylamide/bis acrylamide solution (30% T, 2.6% C)
85 ml deionized water
62.5 ml separating buffer (18.15 g) Tris-HCl in 100 ml water, pH 8.8
625 mg bovine α-casein

To make a zymogram, 10 ml of this solution was combined with 30 µ 1 of 10 % ammonium persulfate and 30 µl of N,N,N',N'-tetra-methylethylene diamine (TEMED), then poured between 10 cm glass plates separated by 0.5mm thick spaces.

The zymogram gel was overlaid with a stacking gel comprised of the following:
1 ml acrylamide/ bis acrylamide solution (30 % T/2.6% C)
2.5 ml stacking buffer (3.0g Tris-HCl in 50 ml water, pH 6.8)
0.1 ml 10% SDS
0.02 ml 10% ammonium persulfate, plus water to a total volume of 10 ml

10µl of TEMED were added to initiate polymerization and the gel was then overlaid on the zymogram gel and allowed to polymerize. Once polymerized, the sample was applied and the electrophoresis was performed to introduce the samples into the zymogram.

Four different buffers are used in the course of running the zymogram. The constituents of these buffers are shown below:

### 1) Tris-Glycine SDS Sample Buffer, (2x) 20ml

| | |
|---|---|
| 0.5M Tris-HCl, pH 6.8 | 2.5ml |
| Glycerol | 2.0ml |
| 10% (w/v) SDS | 4,0ml |
| 0.1% bromophenol blue | 0.5ml |
| Distilled water to | 10.0ml |

### 2) Tris-Glycine SDS Running Buffer (10x)

| | |
|---|---|
| Tris Base | 29g |
| Glycine | 144g |
| SDS | 10g |
| Distilled water to | 1 liter |

### 3) Renaturing Buffer

Triton X-100, 2.5% (v/v) in water

### 4) Developing Buffer

| | |
|---|---|
| Tris Ease | 12.1g |
| Tris HCl | 63.0g |
| NaCl | 117g |
| Brij 35 (Laureth-23 surfactant from ICI Americas) | 0.02% (w/v) |
| Distilled water to | 1 liter |

^{∗} 1x running buffer should be pH 8.3. Do not use acid or base to adjust pH.

To conduct the zymogram, mix one part of the sample with one part 2x Tris-Glycine SDS Sample Buffer and let stand for ten minutes at room temperature. Do not heat.

Apply samples (typically 10-25 µl) and run the gel with 1x Tris-Glycine SDS Running Buffer according to the following running conditions.
- Voltage:: 100V constant
- Current:: Start: 30-40mA/gel End: 8-12mA/gel
- Run Time:: Approximately 90 minutes. The run is complete when the bromophenol blue tracking dye reaches the bottom of the gel.

After running, dilute the Zymogram Renaturing Buffer 1:9 and incubate the gel in the buffer (100 ml for one or two gels) with gentle agitation for 30 minutes at room temperature.

Decant the Zymogram Renaturing Buffer and replace with 1 x Zymogram Developing Buffer (100 ml for one or two gels), Equilibrate the gel for 30 minutes with gentle agitation then replace with fresh 1 x Zymogram Developing Buffer and incubate at 37°C for at least four hours. Incubate overnight for maximum sensitivity. Incubation time can be reduced to as little as one hour for concentrated samples. The optimal result can be determined empirically by varying the sample load or incubation time.

Gel staining is conducted using comassie blue (typically 0.5 to 2.5%) in 40% reagent alcohol, or methanol and 10% acetic acid. Areas of protease activity show up as clear bands.

Destaining is conducted using 40% reagent alcohol and 10% acetic acid, followed by 10% reagent alcohol and 5% acetic acid.

### EXAMPLE 1

Protease extracts were obtained and zymograms were conducted according to the protocol described above, except that the strips were incubated for 36 hours in development buffer that had the pH adjusted to 5, 6, 7, 8, or 9, with or without 1 mM zinc chloride. Incubations were done at 37° C. The gels were stained and destained according to normal practices.

In the samples run without zinc chloride, proteolytic activity was seen at all pH levels. However, at pH's above 6, zinc-induced inhibition of proteolytic activity was seen. These results are consistent with histidine playing a role in inhibition of skin protease, because the pKa of histidine is about 6-6.5. It was also observed that the maximum proteolytic activity occurred at pH 6.

### EXAMPLE 2

The data in Example 1 suggest that there is a histidine at or near the active site of the skin proteases and zinc is capable of chelating with this active site to inhibit the activity of the protease. To further test the evidence that histidine reactive reagents inhibit skin enzymes, additional experiments were conducted.

In this experiment diethyl pyrocarbonate (DEP)and acetic anhydride (AA) were studied in various dilutions, so that the final concentration of the active agents tested were 0, 0.25, 0.5, 1.0, 2,0, 5.0, and 10.0 mM. Both of these compounds are known to modify histidine residues.

The reactions were set up with 20 µl of skin extract and 2 µl of the active agent under investigation (DEP or AA). They were then incubated at 37° C for 30 minutes. All reactions then received 2 µl 10 x SDS page sample buffer and were incubated for another 30 minutes at the same temperature.

The samples were then run on zymograms, developed 12 hours at 37° C and stained and destained.

It was observed that proteolytic activity is suppressed at 0.5 and 1.0 mM DEP and completely abolished at higher concentrations of DEP. Similarly, proteolytic activity was suppressed at AA concentrations of 2.0 and 5.0 mM and conpletely abolished at 10.0 mM.

These results do suggest that histidine is involved in the proteolytic activity of skin enzymes. Although AA and DEP can react with other amino acid residues, they tend to be specific for accessible His residues.

### EXAMPLE 3

In this series of experiments the inhibitory effect of iodine containing compositions and compounds was evaluated.

Povidone, a commercially available iodine containing-composition, was used as the source of iodine in the first experiment. Twofold dilutions of the povidone were made using sterile water.

Reactions were set up using 45 µl of skin extract and 5 µl of Povidone solution. They were incubated 10 minutes at 37°. Then 5 µl 100% glycerol was added and after an additional 10 minutes incubation at 37°C, 35 µl of the mixtures were applied to a zymogram run at 100V.

The zymogram was renatured in renaturing buffer for 30 minutes, then developed overnight at 37°C in 50 um sodium phosphate, pH 6.0.

Proteolytic activity was only seen at the zero treatment control in the zymogram. At the very lowest concentration, 0.0031%, no proteolytic band is seen. Based on these observations, iodine appears to be a potent inhibitor of the action of skin proteases.

Further experiments were run to determine whether the activity seen in the above experiment was due to iodine or components present in the Povidone mixture.

Iodine, available from Sigma Chemicals, was made to 10mM in 100% ethanol from which ten-fold serial dilutions were made. Reactions were set up containing 5 µl of iodine and 45 µl human skin extract. The control consisted of 45 µl extract and 5 µl 100% ethanol.

Each reaction was then given 5 µl glycerol containing 0.5 bromphenol blue and 25 µl were applied to a casein zymogram. The zymogram was run at 100V, then renatured in renaturing buffer for 30 minutes at room temperature. The zymogram was then developed overnight at 37°C in 50 mM sodium phosphate at pH 6.0. The gel was then stained and destained, as previously described.

In this experiment, iodine was completely inhibitory at a final concentration of 1mM, but was not seen to be completely inhibitory at 0.1, 0.01 or lower concentrations. The present inventor concluded that this experiment confirms the previous observation that iodine causes inhibition of the stratum corneum protease and proves that the previously seen inhibition is due to iodine.

To further evaluate the effect of iodine on skin proteases, Povidone was painted onto the skin in areas about 2 inches by four inches prior to extraction with tape. One area on the upper arm was left unpainted and used as an untreated control. Tape strips were taken and extracted with 1% SDS in 50 uM sodium phosphate, pH 6, for 30 minutes at room temperature. The extracts were then applied to a casein zymogram.

After running the gel, it was renatured in renaturing buffer and incubated overnight as previously described. The gel was stained and destained as before.

The gels showed proteolytic activity from the controls. All extracts from the treated areas lacked a band of protease activity. These results appear to confirm the previous finding that Povidone inhibits skin protease.

### EXAMPLE 4

In this series of experiments, the effects on skin proneolytic activity of histidine or sulfhydryl reactive reagents was tested.

Skin extracts were obtained using the method previously subscribed and 1% SDS in 50mM sodium phosphate at pH 3.0. Additionally, porcine skin extracts were evaluated. The following reagents were tested for their inhibitory effect.
Dithiothrietol (DTT)
N-ethyl maleimide (NEM)
Povidone
Chloramine-T
N-acetyl imidazole
Succinic anhydride
N-bromosuccinimide
30% hydrogen peroxide
Ferric chloride

All the above reagents were available from Sigma chemicals, with the exception of the Povidone (Butler) and hydrogen peroxide (Fisher).

Fourteen tape strips were taken and each was extracted with 100 µ l 1% SDS for one hour at room temperature then centrifuged 1000 rpm for five minutes. The eluates were collected and pooled. The following reactions were then set up using either human or porcine extract.
1. 25 µl extract
2. 25 µl extract and 1 µl 0.25 M DTT
3. 25 µl extract and 1 µl 0.25M DTT and 1 µl 0.3
   NEM( added after incubation)
4. 25 µl extract and 1 µl Povidone
5. 25 µl extract and µl 50 mM chloramine T
6. 25 µl extract and 1 µl 50 mM N-acetylimidazole
7. 25 µl extract and 1 µl 50 mM succinic anhydride
8. 25 µl extract and 1 µl 50 mM N-bromosuccinimide
9. 25 µl extract and 1 µl 30% hydrogen peroxide
10. 25 µl extract and 1 µl 30% hydrogen peroxide and 1 µl 25 mM ferric chloride

The reactions were all incubated at 37°C for one hour. Then NEM was added to the third composition above, and all ten samples received glycerol with bromphenol blue. They were applied to the zymogram gel and run in the normal fashion, including renaturing, staining and destaining.

The porcine samples show two protease bands, one of higher molecular weight than the other. The human samples have a single band, corresponding generally in molecular weight and susceptibility to the porcine low molecular weight band. The higher molecular weight porcine band showed different susceptibility than the lower molecular weight bands.

It was observed that reduction, via DTT, eliminates the activity of the lower molecular weight human and porcine proteases, but had no effect on the higher molecular weight protease.

Povidone eliminates the activity of human and porcine low molecular weight enzyme and possibly attenuates the high molecular weight pig enzyme. The same effect was observed for Chloramine T.

N-acetyl imidazole and succinic anhydride do not inhibit the lower molecular weight enzyme and may possibly increase the activity of the higher molecular weight enzyme.

N-bromosuccinimide and the combination of hydrogen peroxide and ferric chloride inhibited all the species. Hydrogen peroxide alone inhibited the lower molecular weight enzyme from both species and appeared to attenuate the activity of the higher molecular weight enzyme.

### EXAMPLE 5

In light of the activity shown in inhibiting the activity of skin proteases by both chloramine T and iodine containing compounds, an experiment was run to determine if a combination of these two agents exhibited a synergistic effect.

Solutions of 10mM chloramine T and 10mM sodium iodide were mixed to produce 5mM chloramine T / 5mM NaI. Tenfold serial dilutions were then made in water. Reactions were then set up using 27 µl skin extract and 3 µl of the chloramine T/ NaI mixture. Zymograms were run as previously described.

The sodium iodide alone was not inhibitory and chloramine T alone was inhibitory at a final concentration of 1mM. The combined agents were inhibitory at a final concentration of 0.5mM each. Based on these observations, it was not possible to separate the effect of NaI and chloramine T from the effect of chloramine T, thus it does not appear that the combination is synergistic.

### EXAMPLE 6

Other histidine modifying reagents were tested for their inhibitory effect. Tape extractions were done in 50mM sodium phosphate at pH 8 and 1% SDS. Reactions were set up with 25 µl of extract as follows.
1 Extract alone
2. 1 µl Povidone
3. 1 µl 1M NaI
4. 1 µl 1M NaI and 1 µl 1M chloramine T
5. 1 µl 1M chloramine T
6. 25 µl iodogen
7. 25 µl iodogen and 1 µl 1M NaI
8. 1 µl lactoperoxidase
9. 1 µl lactoperoxidase and 1 µl hydrogen peroxide (1:4000 of
   30%)
10. 1 µl lactoperoxidase and 1 µl of hydrogen peroxide and 1 µl
   NaI

Zymograms were run as previously described. Iodination by oxidative mechanisms successfully inhibited the protease. Enzymatic iodination was not successful, possibly because lactoperoxidase is a fragile enzyme and the extracts contain 1% SDS. Oxidation without the use of iodine was also successful at inhibiting the protease.

### EXAMPLE 7

Based on the theory that free radical generation by UV light should oxidize the histidine residue at or near the reactive site in the skin protease, experiments were run to determine whether UV light can inhibit skin proteases.

Sixteen tape strips were obtained from the right and left volar forearms as previously described.
A Fotodyne 3-4400 UV light box was calibrated with a UV meter and shown to have the distribution given below in milliwatts/cm2. The average was 4.3 milliwatts/cm2.

| | | | |
|---|---|---|---|
| 2.13 | 3.32 | 3.76 | 1.13 |
| | | | |
| 2.77 | 4.75 | 4.66 | 1.52 |
| | | | |
| 2.28 | 4.68 | 4.62 | 1.79 |

The above chart represents the face of the light box.

As can be seen from the above, the light box gave the strongest readings toward the center and the edges were weaker in strength.

The sixteen tape strips were randomly placed in the light box, with care taken to avoid the edges. The tape strips were then irradiated for 60 seconds. Two tape strips were then removed and the remaining strips were randomly rearranged and irradiated for another 60 seconds. This process was repeated until there were two samples for 0 to seven minutes. Based on the random repositioning it was believed that uniform irradiation was achieved for the samples.

The tape strips were then extracted as previously described and run on zymograms. The zymograms were arranged in two sets to simplify visualization of the UV effects.

In both gels reduction in proteolysis is seen with the increased time of UV irradiation. Some residual activity was seen at seven minutes, which represents 0.8 W minutes. These observations lead to the conclusion that UV light is an inhibitor of skin proteases.

### EXAMPLE 8

Based on previously described observations, see Example 4, which indicated that disulfide reduction abolished skin protease activity, it was decided to determine what concentrations of reducing agents causes loss of caseinolytic activity of skin proteases, as shown in zymograms.

In this experiment, the following compounds were evaluated:
N-acetyl cysteine (Sigma)
Dithiothreitol (Calbiochem)
Cysteine (Sigma)
Human serum albumin (Sigma)

The compounds were made up in dilutions of 25, 10 and 5 mM. Five µl of reducing reagent was mixer with 20 µl of skin extract, which was obtained as previously described. The mixture was then incubated at 37°C for 20 minutes. 10 x SDS PAGE (polyacrylamide gel electrophoresis) sample buffer was added, i.e. 2 µl, and the mixtures were incubated at 37°C for ten minutes before application to the zymograms.

Neither N-acetyl cysteine or human serum albumin had any effect on skin proteases.

DTT produced complete inhibition of the skin protease at a final concentration of 1 and 2.5 mM and partial inhibition at 0.5 mM. Cysteine produced partial inhibition at 2.5 and 1 mM and 0.5 mM appeared to have very little effect on skin protease activity.

The above results confirm that reducing agents do abolish proteolytic activity of skin protease. However, the lack of effect by N-acetyl cysteine suggests that not all sulfhydryl containing compounds will work. This may relate to the accessibility of the internal disulfides within the skin proteases.

In connection with the many types of agent which can be used to inhibit the activity of skin proteases, several methods for employing these agents in conjunction with the transdermal delivery of peptide drugs can be used. One such method involves the direct application of the inhibitory agent directly on the skin for a predetermined length of time prior to the application of the transdermal patch. These agents could be supplied in a sealed foil pouch, with the agent contained in an absorbent pad within the pouch. The patient would then open the pouch and apply the agent by wiping the intended location of the transdermal patch.

Another method would be to incorporate the agent in an absorbent pad at the bottom of the transdermal device, which could be sealed with an outer protective layer until immediately before use. The patch could be applied for a period of time prior to the start of the iontophoresis to permit the inhibitory agent to act on the skin protease.

The most preferred method of delivering the protease inhibiting agent is to incorporate it into the drug reservoir in the transdermal patch. In this manner, the protease inhibiting agent can be delivered simultaneously with the peptide drug, thus helping to ensure that the skin proteases are de-activated throughout the administration of the drug. Agents especially well-suited for this method are zinc salts at a pH of about 7 to about 9, and iodine and/or iodide containing agents.

In the case of the use of UV radiation, a patient would need to have a power supply source sufficient to achieve an inhibitory effect prior to application of the transdermal patch.

While the preferred embodiments of the present invention have been described so as to enable one skilled in the art to practice the present invention, it is to be understood that variations and modifications may be employed without departing from the concept and intent of the present invention as defined in the following claims. The preceding description is intended to be exemplary and should not be used to limit the scope of the invention. The scope of the invention should be determined only by reference to the following claims.

## Claims

1. A method of delivering a peptide drug to a patient via a transdermal patch, wherein the activity of one or more proteases contained in the patient's stratum corneum is inhibited by using an agent which reacts with histidine residues within the proteases.

2. The method according to claim 1 wherein the agent is a zinc salt at a pH above 6.0.

3. The method according to claim 1 wherein the agent contains iodine and/or iodide.

4. The method according to claim 1 wherein the agent causes photooxidation,

5. The method of claim 1 wherein the agent is topically applied to an area of the skin prior to or simultaneously with application of the transdermal patch.

6. The method of claim 1 wherein the transdermal patch is an iontophoretic patch.

7. A transdermal patch for delivering a peptide drug to a patient, said patch including a reservoir for the peptide drug, wherein an agent which reacts with histidine residues of the skin proteases, is incorporated into the drug reservoir

8. The patch according to claim 7 wherein the agent is a zinc salt at a pH of above 6.0.

9. The patch according to claim 7 wherein the agent contains iodine and/or iodide.

10. The patch according to claim 7 wherein the patch is an iontophoretic patch.
